# EUROPÄISCHE PATENTSCHRIFT

(11) **EP 0 507 162 B1**
(45) Veröffentlichungstag und Bekanntmachung des Hinweises auf die Patenterteilung: **09.03.1994**
(21) Anmeldenummer: 92104908.6
(22) Anmeldetag: 20.03.1992
(51) Int. Cl.: A61B 17/58

(54) **Knochenplattenanordnung**
Bone plate device
Plaque ostéosynthétique

(30) Priorität: 03.04.1991 DE 9104025 U
(43) Veröffentlichungstag der Anmeldung: 07.10.1992
(73) Patentinhaber: Waldemar Link (GmbH & Co.), D-22315 Hamburg (DE)
(72) Erfinder: Keller, Arnold, W-2061 Kayhude (DE)
(74) Vertreter: Glawe, Delfs, Moll & Partner

(56) Entgegenhaltungen:
- EP-A- 410 309
- EP-A- 0 077 681
- EP-A- 0 085 493
- EP-A- 0 241 914
- WO-A-90/12547
- DE-A- 3 027 138
- DE-C- 251 246
- GB-A- 2 178 323

## Beschreibung

Es sind Knochenplattenanordnungen, insbesondere für die Wirbelsäulenchirurgie bekannt, die es gestatten, eine feste Winkelrelation zwischen der Knochenschraube und der Knochenplatte herzustellen, beispielsweise um einem Knochenfragment oder einem Wirbelkörper eine vorbestimmte Winkelstellung im Verhältnis zu benachbarten Fragmenten oder Wirbelkörpern zu geben (EP-PS 0 201 024, EP-OS 0 242 842). Dabei ist die Stelle, an der die Knochenschraube durch die Knochenplatte hindurchtritt, durch die Bohrungsanordnung in der Knochenplatte festgelegt. Die bekannten Anordnungen sind auch sehr dick. Ferner kann die Knochenplatte den Blick und den Zugang zu derjenigen Stelle behindern, an der eine Knochenschraube gesetzt werden soll. Schließlich haben manche bekannte Knochenplattenanordnungen den Nachteil, daß der Zusammenhalt zwischen Knochenplatte und Schraube sich löst, wenn die Schraube sich im Knochen lockert, wobei weiter die Gefahr besteht, daß die Schraube durch die Knochenplatte hindurch nach außen herauswandert. Das gilt auch für eine bekannte Anordnung (EP-A-0 410 309), bei der der halbsphärisch ausgebildete Kopf der Schraube in der sphärischen Vertiefung einer Stützplatte ruht, die ihrerseits an einer als Langloch ausgebildeten Durchgangsöffnung der Knochenplatte einstellbar ist und sich in der gewählten Stellung durch zusammenwirkende Riffelung hält.

Bekannt ist ferner eine Knochenplattenanordnung (WO-A-9012547, Fig. 3), bei der die Schraube in der Durchgangsöffnung der Knochenplatte mittels einer besonderen Hülse fixiert wird. Diese besteht aus zwei miteinander verschraubbaren Teile, die jeweils einen sich auf die Unter- bzw. Oberseite der Knochenplatte auflegenden Flansch bilden und einen Klemmkonus einschließen, der auf den Zapfenteil der Knochenschraube einwirkt. Wenn die beiden Hülsenteile so gegeneinander geschraubt sind, daß ihre Flanschen kraftschlüssig an der Knochenplatte fest anliegen, fixiert der Klemmkonus die Knochenschraube. Diese Anordnung, die der Bildung des Oberbegriffs des Anspruchs 1 zugrundeliegt, ermöglicht auch dann eine winkelstabile Fixierung der Schraube gegenüber der Knochenplatte, wenn die Schraube sich im Knochen lockert, wobei das Auswandern der sich lockernden Schraube verhütet wird. Jedoch verlangt die Anordnung hohe Fertigungsgenauigkeit und Montagesorgfalt, weil anderenfalls entweder die Fixierung der Schraube in der Hülse oder die Fixierung der Hülse gegenüber der Knochenplatte unzureichend ist.

Der Erfindung liegt daher die Aufgabe zugrunde, eine Knochenplattenanordnung der im Oberbegriff des Anspruchs 1 genannten Art zu schaffen, die bei unkompliziertem Aufbau große Sicherheit bietet. Ferner will die Erfindung größere Freiheit beim Setzen der Schraube und besseren Zugang zum Knochen ermöglichen.

Dadurch, daß die Hülse unabhängig vom Setzen der Knochenschraube an der Knochenplatte fixierbar ist, läßt sich die Winkellage der Knochenschraube gegenüber der Knochenplatte und/oder die Position der Knochenschraube entlang einem in der Knochenplatte vorgesehenen Langloch unabhängig vom Einbringen der Knochenschraube in den Knochen bestimmen. Diese Ausführung erlaubt eine Vorgehensweise, bei welcher zunächst die Schraube(n) gesetzt wird (werden) und erst danach die Knochenplatte auf die Schraube(n) aufgesetzt wird. Die Platte behindert daher nicht die Zugänglichkeit zum Knochen, wenn die Schraube gesetzt wird. Die Anordnung bleibt in sich als feste Einheit erhalten, wenn eine Schraube sich im Knochen lockert. Insbesondere bleiben die Abstände und die Winkelstellungen von mit derselben Knochenplatte verbundenen Schrauben erhalten. Schließlich ist es auch nicht möglich, daß eine Schraube nach ihrer Lockerung im Knochen aus der Knochenplattenanordnung herauswandert.

Sobald feststeht, an welcher Stelle der Knochenplatte die Schraube mit dieser verbunden werden soll, kann die Hülse fest mit der Knochenplatte verschraubt werden. Nach dem Setzen der Schraube wird dann die Knochenplatte bzw. die daran vorgesehene Hülse auf die Schraube aufgesetzt. Die Hülse kann so ausgebildet sein, daß sie eindeutig winkelfest mit der Platte verbunden werden kann, wobei die Winkelfestigkeit zwischen Knochenschraube und Hülse dadurch gewährleistet ist, daß die Durchgangsöffnung in der Hülse beliebig lang und passend zum Durchmesser des Schraubenschafts ausgeführt werden kann.

Der Gegenflansch der Hülse ist zweckmäßigerweise so ausgebildet, daß er der Knochenplatte eine ebene Fläche zuwendet, durch die die Winkelstellung der Hülse gegenüber der Platte festgelegt ist. Im allgemeinen ist Rechtwinkligkeit erwünscht, so daß der Gegenflansch rechtwinklig zur Hülse verläuft. Wenn eine schräge Anordnung der Schraube zur Knochenplatte beabsichtigt ist, kann stattdessen eine entsprechend winklige Anordnung des Gegenflanschs an der Hülse vorgesehen werden.

Nach einer Ausführungsart der Erfindung ist vorgesehen, daß das zur Aufnahme der Hülse vorgesehene Knochenplattenloch (bzw. Knochenplattenlöcher) mindestens in einer Richtung weiter als der äußere Hülsendurchmesser ist, so daß die Hülse in diesem Loch in mindestens einer Richtung verstellbar ist. Die positive Lokalisierung der Hülse gegenüber der Knochenplatte kann dann durch Klemmung an den Plattenoberflächen erfolgen. Diese wird erfindungsgemäß dadurch verbessert, daß wenigstens eine Seite der Knochenplatte und die damit zusammenwirkende Fläche einer mit der Hülse verbundenen Stützplatte oder des Gegenflansches der Hülse zueinander passend rauh ausgebildet sind. Wenn die Knochenplattenlöcher als Langlöcher ausgebildet sind, wird die Rauhigkeit zweckmäßigerweise als quer zur Langlochrichtung verlaufende Riefelung ausgeführt, um der Verschiebung in Langlochrichtung besseren Widerstand leisten zu können, während die Positionierung quer dazu durch das Langloch selbst erfolgen kann.

Wünscht man den Winkel der Knochenschraube gegenüber der Knochenplatte frei einstellen zu können, so kann nach einer Ausführungsart der Erfindung vorgesehen sein, daß in der mutterseitigen und gegenflanschseitigen Abstützung der Hülse an der Knochenplatte je eine sphärische Stützfläche vorgesehen und wenigstens auf einer Seite die Stützkraft durch eine verschiebbare, über Rauhigkeit an der Knochenplatte anliegende Stützplatte übertragbar ist. Beispielsweise kann, wenn eine freie Positionierung der Knochenschraube in einer Flächenrichtung der Knochenplatte nicht erforderlich ist, das Durchgangsloch in der Knochenplatte mit einer sphärisch konvexen Fläche des Gegenflansches der Hülse zusammenwirken, während auf der anderen Seite die konvex-sphärische Unterfläche der Mutter mit einer Stützplatte zusammenwirkt, die ihrerseits ebenso wie die Knochenplattenoberseite mit einer Rauhigkeit versehen ist. Wenn hingegen eine Verschiebbarkeit der Schraube in einer Flächenrichtung der Knochenplatte erwünscht ist, wirkt die konvex-sphärische Fläche des Gegenflansches nicht unmittelbar mit der Knochenplatte zusammen, sondern mit einer Stützplatte, die ihrerseits mit der Unterseite der Knochenplatte über eine Rauhigkeit zusammenwirkt.

Nach einer weitere Ausführungsart der Erfindung kann vorgesehen sein, daß die Schraube auch noch nach der Verbindung mit der Knochenplatte drehbar ist. Beim Setzen der Schraube kann nämlich u.U. noch nicht genau deren erforderliche Höhe im Verhältnis zur Knochenoberfläche abgeschätzt werden und sie wird daher bewußt weniger weit eingetrieben, als dies voraussichtlich erforderlich scheint. Erst nach der Verbindung der Mutter mit der Knochenplatte wird die Schraube so weit eingedreht, daß die Knochenplatte die gewünschte Lage an der Knochenoberfläche erhält. Dabei kann vorgesehen werden, daß die Knochenplatte nicht unter Druck an der Knochenoberfläche anliegt, wodurch erfindungsgemäß die Lockerungsgefahr weiter vermindert werden kann. Während nämlich bei bekannten Anordnungen die Knochenschraube ständig mit der Gegenkraft beaufschlagt ist, die den Druck der Knochenplatte auf die Knochenoberfläche ausgleicht, wodurch auch im Ruhezustand eine Knochenrückbildung im Bereich der Gewindegänge und damit eine Lockerung verursacht werden kann, ist die erfindungsgemäße Anordnung im Ruhezustand von derartigen Kräften frei.

Die erwähnte Drehbarkeit der Knochenschraube nach ihrer Verbindung mit der Knochenplatte kann dann, wenn die Knochenschraube mittels einer Mutter an der Hülse gesichert wird, dadurch gewährleistet sein, daß das am Bolzenende zur Aufnahme der Mutter vorgesehene Gewinde so kurz bemessen ist, daß eine feste Auflage der Mutter an der Hülse nicht zustande kommt. Zum Drehen der Schraube kann man dann mit einem Werkzeug an der Mutter angreifen. Stattdessen könnte man natürlich auch Schlüsselflächen benutzen, die am Schraubenschaft vorgesehen sind. Jedoch sind derartige Maßnahmen im allgemeinen nicht notwendig, da normalerweise die Schraube auch dann noch jedenfalls in einschraubender Richtung mittels eines an der Mutter angreifenden Werkzeugs drehbar bleibt, wenn die Mutter in vollständige Anlage an der Knochenplatte oder eines mit ihr verbundenen Teils gelangt ist.

Die Knochenplatte kann, wie an sich bekannt, in Querrichtung gewölbt sein, um sich mit Knochenoberflächen entsprechender Wölbung stabiler verbinden zu können.

Die Erfindung wird im folgenden näher unter Bezugnahme auf die Zeichnung erläutert, die vorteilhafte Ausführungsbeispiele veranschaulicht. Darin zeigen:
- Fig. 1: eine Vertikalansicht der an einem Wirbelkörper angebrachten Anordnung,
- Fig. 2: eine Seitenansicht einer ähnlichen Anordnung,
- Fig. 3 und 4: eine Knochenplatte mit einer bzw. zwei Reihen von Langlöchern,
- Fig. 5: eine teilweise geschnittene Seitenansicht der Knochenplattenanordnung,
- Fig. 6: eine Explosionsdarstellung der beteiligten Elemente,
- Fig. 7 und 8: eine winkeleinstellbare Anordnung und
- Fig. 9: eine Draufsicht.

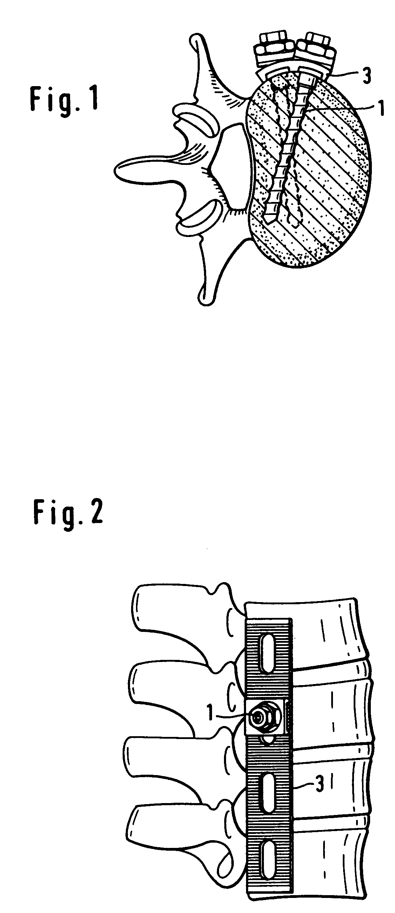

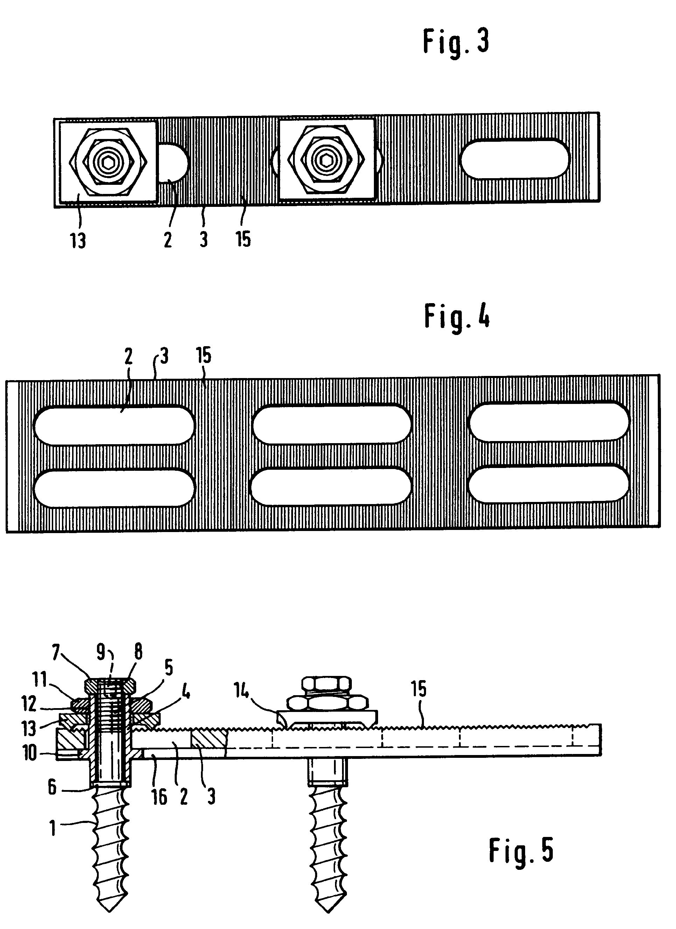

Fig. 1 und 2 zeigen die Anordnung seitlich an eine Reihe von Wirbelkörpern angesetzt, wie es bei der ventralen Wirbelsäulenchirurgie erforderlich sein kann. Die Knochenplatte ist in Querrichtung gewölbt, um sich der Kontur der Wirbel besser anpassen zu können. In anderen Anwendungsfällen mag dies entbehrlich sein. In Fig. 1 ist vorausgesetzt, daß die Knochenplatte - wie in Fig. 4 dargestellt - zwei Reihen von Langlöchern aufweist, um eine versetzte Schraubenanordnung in zwei Schraubenreihen zu ermöglichen, während die in Fig. 2 und 3 dargestellte Knochenplatte lediglich eine Reihe von Langlöchern enthält.

Die Knochenschrauben 1 werden in den Langlöchern 2 der Knochenplatten 3 mittels Hülsen 4 gehalten, deren Bohrung mit dem erforderlichen Spiel dem Durchmesser des Schafts 5 der Knochenschrauben entspricht. Zur Befestigung in den Hülsen sind die Knochenschrauben je mit einem Bund 6 und einer Mutter 7 ausgerüstet, die auf einem auf dem Ende des Schafts 5 angeordneten Gewinde 8 sitzt. Die Knochenschraube besitzt, um unabhängig gedreht werden zu können, am Ende einen Innensechskant 9.

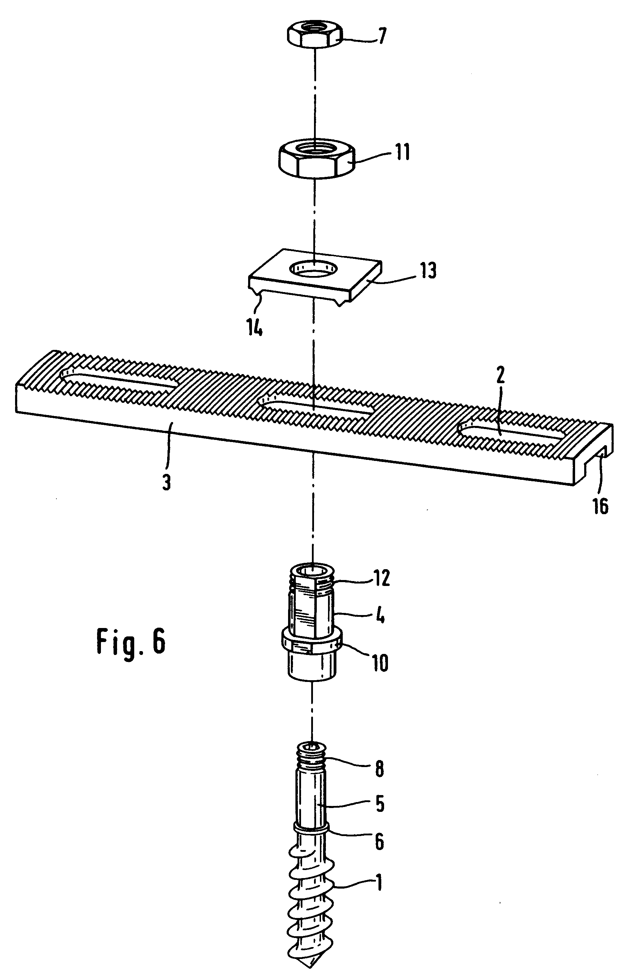

Die Hülse weist zur Verbindung mit der Knochenplatte 3 einen Gegenflansch 10 sowie eine Mutter 11 auf einem Gewinde 12 auf. Die Mutter 11 stützt sich über eine Stützplatte 13 an der Oberseite der Knochenplatte 3 ab, die zwei quer zur Zeichenebene der Fig. 5 verlaufende, schneidenförmige Erhöhungen 14 an ihrer Unterseite hat. Die Oberseite der Knochenplatte ist quer zur Richtung der Langlöcher 2 gerieft, wie bei 15 angedeutet ist, wobei die Schneiden 14 in die Riefen 15 eingreifen, wodurch die Stützplatte 13 an einer Verschiebung in Richtung der Langlöcher 2 gehindert ist, wenn die Mutter 11 angezogen ist. Diese Schneiden bzw. Riefen bilden die weiter oben erwähnte Rauhigkeit. Dabei stützt sich der Gegenflansch 10 an der Unterseite der Knochenplatte 3 ab, wobei er innerhalb einer Nut 16 liegt, die ihn passend aufnimmt. Die Hülse 4 und der Gegenflansch 10 sind, wie besser in Fig. 6 erkennbar ist, seitlich abgeflacht, wobei die Abflachungen 17 mit den Flanken der Langlöcher 2 bzw. der Nut 16 zusammenwirken, um die Hülse 4 an Drehung zu hindern, wenn die Mutter 11 angezogen oder gelöst wird.

Für das Vorgehen bei der Operation ist kennzeichnend, daß zunächst die Schrauben gesetzt werden, wobei die Knochenplatte (mit oder ohne daran befestigte Hülsen) in der Art einer Bohrlehre benutzt werden kann. Für das winkelgerechte Vorbohren kann ein Führungsinstrument vorgesehen sein. Statt dessen können an den vorgesehenen Stellen auch an der Knochenplatte angebrachte Hülsen 4 als Führung für das weitere winkelgerechte Vorbohren dienen, wenn die Winkelstellung des Knochenfragments oder Wirbels gegenüber der Platte nicht verändert werden soll.

Anschließend wird über den Schaft 5 der eingeschraubten Knochenschraube das Loch im Wirbelkörper mittels eines Hohlfräsers weiter aufgebohrt zur Aufnahme des knochenseitigen Endes der Hülse 4. Gleichzeitig kann der Tiefensitz der Knochenschraube im Wirbelkörper kontrolliert werden.

Wenn dies noch nicht geschehen ist, werden nun die Hülsen mit der Knochenplatte verbunden. Danach wird die Knochenplatte mit den Hülsen auf die Schäfte der in den Knochen eingesetzten Schrauben gesetzt bzw. gesteckt und werden die Schrauben mit den Muttern 7 gesichert. Die Verbindung der Hülsen 4 mit der Knochenplatte kann (aber muß nicht) in diesem Stadium noch lose sein, um eine Längenkorrektur (Distraktion, Extraktion) mittels der eingesetzten Knochenschrauben zu ermöglichen. Ein besonderes Instrument kann für diese Zwecke vorgesehen sein. Danach werden die Muttern 11 fest angezogen. Damit ist eine winkelstabile Absicherung der Knochenschrauben, auch bei eventueller Lockerung im Knochen, erreicht. Abschließend werden dann die Schrauben 1 durch Angriff eines Werkzeugs an den Muttern 7 oder an dem Innensechskant 9 so ggfs. weiter in den Knochen eingeschraubt, bis die Knochenplatte 3 an der Knochenoberfläche anliegt.

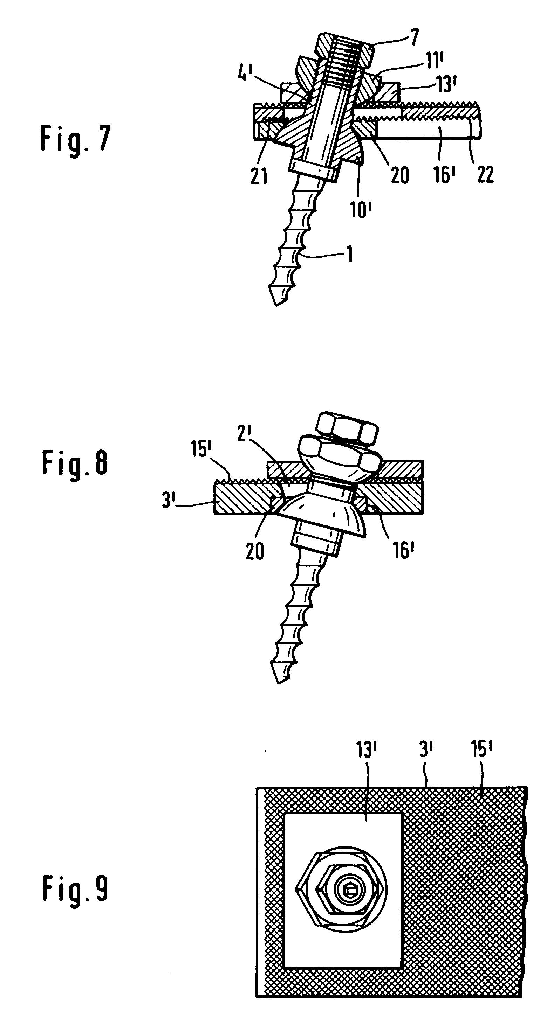

Die Fig. 7 und 8 zeigen einen Längs- und Querschnitt durch eine ähnliche Anordnung, die auch eine Schrägstellung der Schrauben zur Knochenplatte gestattet. Der Gegenflansch 10' der Hülse 4' ist auf der dem Knochen abgewandten Seite konvex-sphärisch ausgebildet, wobei die Sphärenmittelpunkte großen Abstand voneinander haben. Dasselbe gilt gegensinnig für die Mutter 11'. Der Gegenflansch 10' wirkt zusammen mit einer Druckplatte 20, die in der Nut 16' auf der Unterseite der Knochenplatte in Längsrichtung verschiebbar ist und zwei Schneiden 21 (analog den Schneiden 14 der Stützplatte 13) trägt, die mit einer Querriefelung 22 am Grunde der Nut 16' zusammenwirken. Die sphärische Fläche der Mutter 11' wirkt zusammen mit einer entsprechenden, konkav-sphärischen Fläche der Stützplatte 13'. Die Unterseite der Stützplatte 13' und die Oberseite 15' der Knochenplatte sind aufgerauht, und zwar - wie Fig. 9 zeigt - durch quer zueinander verlaufende Riefelungen, so daß sich eine Vielzahl diskreter Pyramidenerhöhungen bildet. Dadurch kann die Stützplatte 13' nicht nur in Längs-, sondern auch in Querrichtung gegenüber der Knochenplatte 3' versetzt und durch Anziehen der Mutter 11' fixiert werden. Demgegenüber kann die Druckplatte 20 in der Nut 16' lediglich in Längsrichtung verschoben werden. Das Loch 2' in der Knochenplatte 3' ist nicht nur in Längsrichtung, sondern auch in Querrichtung weiter als der Außendurchmesser der Hülse 4' ausgeführt. Zusätzlich zu dem Versatz und der Abwinkelung der Schraube in einer Längsschnittebene (Fig. 7) kann daher auch in einer Querschnittsebene (Fig. 8) eine Winkelverstellung vorgenommen werden.

Die Handhabung stimmt mit der oben erläuterten im wesentlichen überein.

## Patentansprüche

1. Knochenplattenanordnung bestehend aus einer Knochenplatte (3) mit wenigstens einer Durchgangsöffnung (2), einer in die Durchgangsöffnung einzusetzenden Knochenschraube (1), und einer einen Zapfen (5) der Knochenschraube aufnehmenden und in der Durchgangsöffnung (2) fixierenden Hülse (4, 4'), die an ihrem einen Ende ein Gewinde (12) zur Aufnahme einer sich an der einen Seite der Knochenplatte (3, 3') abstützenden Mutter (11, 11') und nahe ihrem anderen Ende einen Gegenflansch (10, 10') zur Abstützung an der anderen Seite der Knochenplatte (3, 3') aufweist, dadurch gekennzeichnet, daß die Hülse (4, 4') gesondert von der in ihr aufgenommenen Knochenschraube (1) in der Knochenplatte (3) fixierbar ist beziehungsweise daß die Knochenschraube (1) in die in der Knochenplatte (3) bereits fixierte Hülse (4, 4') aufnehmbar ist, und die Knochenschraube (1) zwischen ihrem in der Bohrung der Hülse (4, 4') aufgenommenen Zapfen (5) und dem Knochengewinde einen Bund (6) und am bundfernen Ende des Zapfens (5) ein Sicherungsglied (7, 8) aufweist.

2. Knochenplattenanordnung nach Anspruch 1, dadurch gekennzeichnet, daß die Hülse (4, 4') in einer vorbestimmten Winkellage in der Knochenplatte fixierbar ist und die Richtung der Knochenschraube (1) mit der Richtung der die Knochenschraube aufnehmenden Hülsenbohrung übereinstimmt.

3. Knochenplattenanordnung nach Anspruch 2, dadurch gekennzeichnet, daß der Gegenflansch (10) eben zur Bestimmung der Winkelstellung der Hülse (4, 4') gegenüber der Platte (3, 3') ausgebildet ist.

4. Knochenplattenanordnung nach einem der Ansprüche 1 bis 3, dadurch gekennzeichnet, daß die Durchgangsöffnung (2) als Langloch ausgeführt ist und wenigstens eine Seite der Knochenplatte (3, 3') und die damit zusammenwirkende Fläche einer mit der Hülse (4, 4') verbundenen Stützplatte (13, 13') zueinander passend rauh ausgebildet sind.

5. Knochenplattenanordnung nach einem der Ansprüche 1 bis 4, dadurch gekennzeichnet, daß in der mutterseitigen und gegenflanschseitigen Abstützung der Hülse (4') an der Knochenplatte (3') je eine sphärische Stützfläche vorgesehen und wenigstens auf einer Seite die Stützkraft durch eine verschiebbare, über Rauhigkeit an der Knochenplatte anliegende Stützplatte (11', 20) übertragbar ist.

6. Knochenplattenanordnung nach Anspruch 1, dadurch gekennzeichnet, daß das Sicherungsglied von einem Gewinde (8) am Ende des Bolzens (5) und einer Mutter (7) gebildet ist.

7. Knochenplattenanordnung nach Anspruch 6, dadurch gekennzeichnet, daß die Knochenschraube (1) mittels der Mutter (7) drehbar ist.

## Claims

1. A bone plate assembly comprising a bone plate (3) with at least one opening (2), a bone screw (1) fitting into the opening and a bushing (4, 4') accommodating a shank (5) of the bone screw and to be fixed in the opening (2), said bushing having at one end a thread (12) to receive a nut (11, 11') bearing against one side of the bone plate (3, 3') and near its other end a counterflange (10, 10') to bear against the other side of the bone plate (3, 3'), characterised in that the bushing (4, 4') can be fixed in the bone plate (3) separately from the bone screw (1) accommodated therein, and/or in that the bone screw (1) can be accommodated in the bushing (4, 4') already fixed in the bone plate (3), and the bone screw (1) has a collar (6) between the shank (5) accommodated in the bore of the bushing (4, 4') and the bone thread and it has a securing member (7, 8) at the end of the shank (5) remote from the collar.

2. A bone plate assembly according to Claim 1, characterised in that the bushing (4, 4') can be fixed at a predetermined angular position in the bone plate and the direction of the bone screw (1) corresponds to the direction of the bushing bore accommodating the bone screw.

3. A bone plate assembly according to Claim 2, characterised in that the counterflange (10) is formed flat so as to determine the angular position of the bushing (4, 4') relative to the plate (3, 3').

4. A bone plate assembly according to any one of Claims 1 to 3, characterised in that the opening (2) is in the form of a slot and at least one side of the bone plate (3, 3') and the surface of a support plate (13, 13') cooperating therewith and connected with the bushing (4, 4') are formed rough to match one another.

5. A bone plate assembly according to any one of Claims 1 to 4, characterised in that a respective spherical supporting surface is provided in the support for the bushing (4') against the bone plate (3') on the nut side and on counterflange side.

6. A bone plate assembly according to Claim 1, characterised in that the securing member is formed by a thread (8) at the end of the shank (5) and a nut (7).

7. A bone plate assembly according to Claim 6, characterised in that the bone screw (1) can be rotated by means of the nut (7).

## Revendications

1. Système à plaque d'ostéosynthése, se composant d'une plaque d'ostéosynthèse (3) comportant au moins une ouverture de passage (2), d'une vis à os (1) pouvant être insérée à travers cette ouverture de passage et d'une douille (4, 4') qui reçoit et fixe dans l'ouverture de passage (2) un téton long (5) de la vis à os et qui présente, à l'une de ses extrémités, un filetage (12) pour recevoir un écrou (11, 11') prenant appui sur l'une des faces de la plaque d'ostéosynthèse (3, 3') et, à proximité de son autre extrémité, une contre-bride (10, 10') destinée à prendre appui sur l'autre face de la plaque d'ostéosynthèse (3, 3'), caractérisé en ce que la douille (4, 4') peut être fixée dans la plaque d'ostéosynthèse (3) indépendamment de la vis à os (3) qui y est reçue, c'est-à-dire que la vis à os (1) peut être reçue dans la douille (4', 4') déjà fixée dans la plaque à os (3), et en ce que la vis à os (1) présente, entre son téton long (5) reçu dans le trou de la douille (4, 4') et le filetage à os, un collet (6) et, à l'extrémité du téton long (5) opposée au collet, un élément formant frein d'écrou (7, 8).

2. Système à plaque d'ostéosynthèse selon la revendication 1, caractérisé en ce que la douille (4, 4') peut être fixée dans la plaque d'ostéosynthèse dans une position angulaire prédéterminée, et en ce que la direction de la vis à os (1) coïncide avec la direction du trou de la douille qui reçoit la vis à os.

3. Système à plaque d'ostéosynthèse selon la revendication 2, caractérisé en ce que la contre-bride (10) est réalisée avec une surface plane pour déterminer la position angulaire de la douille (4, 4') par rapport à la plaque (3, 3').

4. Système à plaque d'ostéosynthèse selon l'une quelconque des revendications 1 à 3, caractérisé en ce que l'ouverture de passage (2) est réalisée sous forme de trou oblong, et en ce qu'une face au moins de la plaque d'ostéosynthèse (3, 3') et la surface, coopérant avec celle-ci, d'une plaque d'appui (13, 13') accouplée à la douille (4, 4') sont réalisées avec des rugosités qui s'adaptent l'une dans l'autre.

5. Système à plaque d'ostéosynthèse selon l'une quelconque des revendications 1 à 4, caractérisé en ce qu'il est prévu une surface d'appui sphérique dans chacune des portées de la douille (4') sur la plaque d'ostéosynthèse (3'), du côté écrou et du côté contre-bride, et en ce que, d'un côté au moins, la force portante peut etre transmise par une plaque d'appui mobile (11', 20) qui est appliquée contre la plaque d'ostéosynthèse par des rugosités.

6. Système à plaque d'ostéosynthèse selon la revendication 1, caractérisé en ce que l'élément formant frein d'écrou est constitué par un filetage (8) à l'extrémité du téton long (5) et par un écrou (7).

7. Système à plaque d'ostéosynthèse selon la revendication 6, caractérisé en ce qu'on peut faire tourner la vis à os (1) au moyen de l'écrou (7).
